# EUROPEAN PATENT SPECIFICATION

(11) **EP 1 068 297 B1**
(45) Date of publication and mention of the grant of the patent: **08.02.2006**
(21) Application number: 99917887.4
(22) Date of filing: 29.03.1999
(51) Int. Cl.: C12N 5/06, C12N 5/08, A61K 35/14, A61K 39/00

(54) **USE OF MONOCYTES DERIVED CELLS, ANTIGENS AND ANTIBODIES FOR OPTIMAL INDUCTION OF IMMUNOTHERAPEUTIC EFFICIENCY**
ANWENDUNG VON MONOCYTE-ABSTAMMENDE ZELLEN, ANTIGENE UND ANTIKÖRPER ZUR OPTIMALEN INDUZIERUNG EINER IMMUNTHERAPEUTISCHEN LEISTUNGSFÄHIGKEIT
UTILISATION DE CELLULES DERIVEES DE MONOCYTES, D'ANTIGENES ET D'ANTICORPS AFIN D'INDUIRE UNE EFFICACITE IMMUNOTHERAPEUTIQUE OPTIMISEE

(30) Priority: 09.04.1998 EP 98400883
(43) Date of publication of application: 17.01.2001
(73) Proprietor: I.D.M. IMMUNO-DESIGNED MOLECULES, 75011 Paris (FR)
(72) Inventor: BARTHOLEYNS, Jacques, F-91440 Bures-sur-Yvette (FR); FOURON, Yves, Marlborough, MA 01752 (US); ROMET-LEMONNE, Jean-Loup, F-75003 Paris (FR)
(74) Representative: Grosset-Fournier, Chantal Catherine
(86) International application number: PCT/EP1999/002103
(87) International publication number: WO 1999/053026

(56) References cited:
- WO-A-87/00054
- WO-A-91/05871
- WO-A-92/05793
- WO-A-94/26875
- WO-A-96/22781
- WO-A-97/10002
- WO-A-97/44441
- MAYORGA L.SL ET AL: "Reconstitution of endosomal proteolysis in a cell-free system" JOURNAL OF BIOLOGICAL CHEMISTRY, vol. 264, no. 10, 1989, pages 5392-5399, XP002077582
- "Biologie Moléculaire de la cellule (third edition)" FLAMMARION EDITION * page 622 *

## Description

The invention relates to the use of monocytes derived cells, antigens and antibodies for optimal induction of immunotherapeutic efficiency.

Macrophages play a major role in the antitumoral response, and they are able to be activated by immunological activators against cancer cells (Adams D. and Hamilton T. : "Activation of macrophages for tumor cell kill. : effector mechanism and regulation" ; in Heppner & Fulton (eds), Macrophages and cancer. CRC Press, 1988, p. 27 ; Fidler M. Macrophages and metastases. A biological approach to cancer therapy. Cancer Res. 45: 4714, 1985).

Furthermore, macrophages, or other cells derived from monocytes or from their precursors, with their strong capacity for endocytosis, digestion, and surface antigen presentation, are capable of inducing a specific immune response.

Macrophages represent the first natural line of defense against infectious agents (bacteria, virus) which are normally killed. However, resistant pathogens can develop ways to escape recognition and killing by macrophages. Protection cannot be very effectively achieved with the attenuated infectious agents or with their recombinant peptidic components.

Monocytes derived cells (MDCs) are immune cells such as obtained by culture of blood mononuclear cells in non adherent gas permeable plastic or Teflon bags for 5 to 10 days at 37°C in O₂/CO₂ atmosphere. Their culture medium (RPMI, IMDM, AIM5 (Gibco) or X-VIVO (Biowhittaker)) contains eventually cytokines or ligands as defined in patents n° PCT/EP93/01232, n° WO94/26875, EP 97/02703 or WO 97/44441 or in the articles mentioned below :
- "Autologous lymphocytes prevent the death of monocytes in culture and promote, as do GM-CSF, IL-3 and M-CSF, their differentiation into macrophages". (Lopez M., Martinache Ch., Canepa S., Chokri M., Scotto F., Bartholeyns J. ; J. of Immunological Methods, 159: 29-38, 1993);
- "Immune therapy with macrophages : Present status and critical requirements for implementation" (Bartholeyns J., Romet-Lemonne J-L., Chokri M., Lopez M. ; Immunobiol., 195: 550-562, 1996) ;
- *"In vitro* generation of CD83⁺ human blood dendritic cells for active tumor immunotherapy" (Thurnher M., Papesh C., Ramoner R., Gastlt G. and al. ; Experimental Hematology, 25: 232-237, 1997) ;
- "Dendritic cells as adjuvants for immune-mediated resistance to tumors" (Schuler G. and Steinman R. M. ; J. Exp. Med., 186: 1183-1187, 1997).

All these patents applications and articles are included herein for references.

They can be activated by INF-γ at the end of culture to obtain in particular cytotoxic macrophages. They can be centrifuged to be concentrated and purified before resuspension in isotonic solution.

Monocytes derived cells (MDCs) can either be killer macrophages, phagocytozing cells, growth factors and cytokines releasing cells, or dendritic cells according to their conditions of differentiation. Dendritic cells can for example be obtained as described in *"In vitro* generation of CD83⁺ human blood dendritic cells for active tumor immunotherapy" (Thurnher M., Papesh C., Ramoner R., Gastlt G. and al. ; Experimental Hematology, 25: 232-237, 1997) and "Dendritic cells as adjuvants for immune-mediated resistance to tumors" (Schuler G. and Steinman R. M.; J. Exp. Med., 186: 1183-1187, 1997), and EP n° 97/02703.

Mature dendritic cells are very potent antigen presenting cells to initiate an immune response. The dendritic cells can be characterized by the induction of T cell proliferation and by their phenotype (presence of CD80, CD86, CD83, MHC-I, MHCII on their membranes).

A bispecific agent could be employed to target antigens (WO 92/05793) and human IgM or IgG antitumoral antibodies are developed to target tumors or metastases. Clinical anti-cancer responses achieved with these monoclonal human antibodies are not impressive due to unsolved problems of tissue distribution and disseminated sites, limited access to the tumor or to low local concentration, inadequate pharmacokinetics or ineffective dosage, and mainly to the lack of effector cells impairing cellular and humoral immune responses against the relevant tumor.

IgG, IgA and IgM antibodies directed against infectious agents can be isolated in human plasma an are prepared as monoclonal antibodies. These antibodies do very seldom neutralize the infection as such ; a cellular immune response is required. Therapeutic vaccines are therefore difficult to develop with attenuated infectious agents or with their recombinant peptidic components.

Pan carcinomic tumor antigens (present in breast, prostate, lung, melanoma, glioma, neuroblastoma and corectal tumors for example) are described and can be targeted, at least *in vitro*, by specific human monoclonal antibodies. These pan antitumor antibodies do not react with normal cells and tissues such as epithelium, fibroblasts, neuroectodermal or muscle cells. However, these antibodies bind through their Fc part to reticuloendothelial cells such as macrophages bearing Fc receptors. Therefore most antibodies do not effectively reach the tumor target antigen.

The inefficiency of anti-tumor or anti-infection antibodies is overcome by the present invention which enables to enhance cellular and humoral anti-tumor and anti-infection immune response by initiating *ex vivo* and controlling the interaction between target antigens, specific antibodies and effector monocyte derived cells.

One aim of the invention is to provide a process for preparing monocytes derived cells presenting antigenic epitopes on their membrane.

The invention relates in a general embodiment to a process for the preparation of monocytes derived cells, in a purified form and substantially free of contaminants, presenting antigenic epitopes on their membranes after interiorization and processing of at least an antigen-antibody complex formed between an antigen and a non-bispecific antibody, under appropriate conditions, said epitopes corresponding to proteolytic degradation products of said antigen,
- with said non-bispecific antibody being directed against a tumor or against an infectious agent, and
- with said antigen being fragments of tumor or of infectious agent, including membranes or tumor apoptotic bodies, or infectious antigens or being recombinant tumor or infectious antigens.

The present invention describes an *ex-vivo* method to favor and control the interaction between autologous macrophages or monocytes derived antigen presenting cells, antibodies such as pan anti-tumor antibodies and relevant tumor antigen(s). This controlled interaction (e.a. tumor Ag-pan anti-tumor Ab-effector macrophage) allows a synergistic enhancement of the relevant biological activities and as a result induces in *vivo* cellular and humoral anti-tumor or anti-infection immune response. Tumor apoptotic bodies are particularly adequate as source of tumor antigen.

The invention takes advantage of an antigen, particularly the availability of the pan tumor antigen, or the tumor apoptotic body as source of tumor antigen, and of the corresponding specific human antibody, to create *in vitro* a molecular complex which is readily internalized by monocytes derived macrophages or antigen presenting cells. The endocytosis of the immune complex mainly via Fc receptors of the macrophages allows effective processing *in vitro.* The invention enables also the favored *ex vivo* interaction between fragments of a patient's tumor membrane, IgM monoclonal antibodies recognizing tumor epitopes on these membranes, and autologous macrophages, resulting in adequate processing of the tumor.

In the case of cancer, by way of example, the immune response is forced in the present invention by initial *in vitro* interaction between the complex pan tumoral Ag-antitumoral Ab-autologous monocytes derived antigen presenting cells or macrophages. This interaction allows endocytosis of the carcinoma antigen or of the apoptotic body via Fc receptors, adequate processing, interaction with MHCI and MHCII molecules and presentation of antigenic tumor peptides on the cell membrane. The tumor epitopes presented on the membrane of the effector cells are recognized by autologous T lymphocytes of the patient. The pan tumoral nature of the antigen avoids the MHC restriction which limits the use of purified tumor antigens/epitopes to a few selected patients. The injection of this preparation to patients with most types of carcinomas induces potent specific humoral and cellular immune responses against the relevant tumor.

In the case of infection, an alternative approach to induce effective immunotherapy is to inject a ternary complex formed between the antibody, the infectious agent and macrophages processing the complex, presenting adequately epitopes of the pathogen and forcing the induction of a long lasting immunity against the pathogen and against subsequent infections.

The expression "purifed form" means that the ternary complex formed by tumor or infectious antigens, antitumor or anti-infectious IgM, IgA, IgG antibodies, and effector macrophages or monocyte derived cells is segregated from other cell types or complexes.

The expression "tumor apoptotic bodies" refers to microbodies isolated from tumor cells or cell lines induced to apoptosis, containing mitochondria and DNA within a membrane exposing tumor antigens (Bellone M., Iezzi G., Roverel Galati G. et al. - "Processing of engulfed apoptotic bodies yields T cell epitopes", Journal of Immunology, 159: 5391-5399, 1997).

The expression "substantially free of contaminants" means that macrophages or monocyte derived cells are 90 % pure and that no contaminants other than the antibody and the tumor Ag or apoptotic bodies or infectious antigens are present.

The expression "antigen-antibody complex formed between an antigen and an antibody under appropriated conditions" means that antigen and corresponding antibody associate by high affinity (10⁴ to 10¹¹ L/M) non covalent binding, the antigen can be particular as in the case of apoptotic bodies, increasing the avidity of the complex formed.

The expression "epitopes corresponding to proteolytic degradation products of said antigen" means that the antigen phagocytosed in vacuoles of macrophages or monocyte derived cells is digested by proteases into smaller polypeptide fragments.

The monocyte derived cells of the invention can be identified by the following method : presence of Fcγ receptors (CD16, CD32, CD64), of Fcµ and Fcγ receptors (CD89) and of CR3, of CR5 and of mannose receptor on their membranes at the same time as high levels of MHCI and of MHCII molecules.

The invention also relates to a process for the preparation of monocyte derived cells presenting antigenic epitopes on their membranes after interiorization and processing of at least an antigen-antibody complex, formed between an antigen and an antibody, said antibody being exclusively a non-bispecific antibody and said process comprising the following steps :
a) preparation of the monocytes derived cells according to the following method:
   1) recovery of blood derived mononuclear cells directly from blood apheresis or from blood bag collection, followed if necessary by centrifugation, to eliminate a substantial part of red blood cells granulocytes and platelets, and collection of peripheral blood leukocytes ;
   2) washing peripheral blood leukocytes obtained at the preceeding steps for instance by centrifugation (to remove 90 % of platelets, red blood cells and debris) to obtain mononuclear cells ;
   3) resuspension of the total mononuclear cells obtained at the preceeding step in culture medium (AIMV, RPMI or IMDM type) at 10⁶ to 2.10⁷ cells/ml, possibly completed by cytokines and/or autologous serum, and culture for 5 to 10 days at 37°C under O₂/CO₂ atmosphere in hydrophobic gas permeable bags, to obtain monocyte derived cells and contaminating lymphocytes;
b) addition of antigens and antibodies to the monocyte derived cells obtained at the preceeding step to form a ternary complex between monocyte derived cells, an antigen and an antibody ;
c) incubation of said ternary complex for a time and at a temperature sufficient to allow endocytosis into intracellular vacuoles of the monocyte derived cells and processing of the antigen-antibody complex, with said processing consisting of digestion of the antigen-antibody complex and association of the epitopes of the antigen resulting from the digestion with MHC molecules, to obtain monocyte derived cells presenting antigenic epitopes on their membranes.

The preparation of the monocyte derived cells can be carried out as described in patents n° PCT/EP93/01232, n° WO94/26875, EP 97/02703 or WO 97/44441 or in the articles mentioned below :
- "Autologous lymphocytes prevent the death of monocytes in culture and promote, as do GM-CSF, IL-3 and M-CSF, their differentiation into macrophages". (Lopez M., Martinache Ch., Canepa S., Chokri M., Scotto F., Bartholeyns J. ; J. of Immunological Methods, 159: 29-38, 1993);
- "Immune therapy with macrophages : Present status and critical requirements for implementation" (Bartholeyns J., Romet-Lemonne J-L., Chokri M., Lopez M. ; Immunobiol., 195: 550-562, 1996) ;
- *"In vitro* generation of CD83⁺ human blood dendritic cells for active tumor immunotherapy" (Thurnher M., Papesh C., Ramoner R., Gastlt G. and al.; Experimental Hematology, 25: 232-237, 1997) ;
- "Dendritic cells as adjuvants for immune-mediated resistance to tumors" (Schuler G. and Steinman R. M. ; J. Exp. Med., 186: 1183-1187, 1997).

According to an advantageous embodiment of the invention, the process is such that
- said antibody is directed against a tumor or against an infectious agent,
- with said antigen is fragments of tumor or of infectious agent, including membranes or tumor apoptotic bodies, or purified tumor or infectious antigens or is a recombinant tumor or infectious antigen.

According to an advantageous embodiment of the invention, in the step of addition, said antigens and antibodies are in the form of a complex.

According to an another embodiment of the invention, in the step of addition, said antigens and antibodies are not in the form of a complex.

According to an advantageous embodiment of the invention, the process comprises the additional following step :
d) centrifugation of the monocyte derived cells presenting antigenic epitopes on their membranes, washing and resuspension, for instance in isotonic medium, to obtain a suspension of the above defined monocyte derived cells.

According to an another advantageous embodiment of the invention, in the process above defined, the step of centrifugation is followed by
e) freezing at temperature below or equal to -80°C aliquots of the above said suspension, with the addition of a cryopreservative.

According to an another advantageous embodiment of the process, the step of freezing is followed by
f) melting said above frozen aliquots at a temperature enabling to obtain a suspension of monocyte derived cells presenting antigenic epitopes on their membranes, for instance at 4°C, washing said suspension and resuspending it, for instance in an isotonic medium, to obtain a suspension of monocyte derived cells presenting antigenic epitopes on their membranes.

Advantageously, in the process of the invention, the antibodies are human or humanised IgG, IgA or preferably IgM (for induction of primary immune response) directed against tumor antigens or against infectious antigens (viral or bacterial).

Advantageously, in the process of the invention, the antigens are purified tumor, viral or bacterial antigens (polypeptides, glycopeptides, oligosacharide) or membrane fragments serving as complex antigens.

Advantageously, in the process of the invention, the antigens are pan tumor antigens present on different tumor types.

The invention also relates to a ternary complex in a purified form, and substantially free of contaminants, between monocytes derived cells, an antigen and an antibody
. with said antibody being exclusively a non-bispecific antibody and being directed against a tumor or against an infectious agent,
. with said antigen being fragments of tumor or of infectious agent including membranes or tumor apoptotic bodies, or purified tumor or infectious antigens or being recombinant tumor or infectious antigens, and
. with said antigen and said antibody being liable to form an antigen-antibody complex under appropriate conditions.

The expresssion "ternary complex" designates a complex formed *ex vivo* between an antigen (tumor or infections origin), an antibody recognizing that antigen and a macrophage or monocyte derived cell binding the previous binary complex on its membrane before internalization.

A ternary complex can be identified by electron microscopy or by FACS analysis (fluorescence cell analysis) of the monocyte derived cell and of the molecules expressed on its membrane. High affinity binding between the antigen and the antibody occurs before binding of the complex to the monocyte derived cell membrane. Binding occurs at a temperature below 10°C while internalization occurs only at a temperature above 20°C.

The expression "purified form" means that the ternary complex formed by tumor or infectious antigens, anti-tumor or anti-infectious IgM, IgA, IgG antibodies, and effector macrophages or monocyte derived cells is segregated from other cell type or complexes.

The expression "substantially free of contaminants" means that macrophages or monocyte derived cells are 90 % pure and that no contaminants other than the antibody and the tumor or infectious antigens are present.

A process for the preparation of a ternary complex between monocyte derived cells, an antigen and an antibody as above defined, comprising the following steps :
1) recovery of blood derived mononuclear cells directly from blood apheresis or from blood bag collection, followed if necessary by centrifugation, to eliminate a substantial part of red blood cells granulocytes and platelets, and collection of peripheral blood leukocytes ;
2) washing peripheral blood leukocytes obtained at the preceeding steps for instance by centrifugation (to remove 90 % of platelets, red blood cells and debris) to obtain mononuclear cells ;
3) resuspension of the total mononuclear cells obtained at the preceeding step in culture medium (AIMV, RPMI or IMDM type) at 10⁶ to 2.10⁷ cells/ml, possibly completed by cytokines and/or autologous serum, and culture for 5 to 10 days at 37°C under O₂/CO₂ atmosphere in hydrophobic gas permeable bags, to obtain monocyte derived cells and contaminating lymphocytes ;

b) addition of antigens and antibodies to the monocyte derived cells obtained at the preceeding step to form of a ternary complex between monocyte derived cells, an antigen and an antibody.

In the process above defined, said antigens and antibodies are advantageously in the form of a complex.

In another embodiment of the process above defined, said antigens and antibodies are not in the form of a complex.

The invention also relates to a ternary complex between monocyte derived cells, an antigen and an antibody such as obtained according to the process above defined.

The invention also relates to a pharmaceutical composition containing as active substance a ternary complex according to the invention, in association with a pharmaceutically acceptable vehicle.

The pharmaceutical compositions of the invention are advantageously in the form of sterile injectable preparations.

The monocyte derived cells are administered at a dose of about 10⁶ to about 10⁹ cells/kg of body weight, particularly from about 10⁷ to about 10⁸ cells/kg of body weight.

The invention also relates to a vaccine containing as active substance a ternary complex according to the invention, in association with a pharmaceutically acceptable vehicle.

The invention also relates to a ternary complex according to the invention, for the preparation of a medicament for treating cancer or infectious diseases.

### EXAMPLES

Macrophages or monocyte derived cells of the following examples are prepared according to the methods described in the above-mentioned references.
1) SCID mice are inoculated subcutaneously with human tumor cells. They are also "humanized" by i.v. injection of about 10 million human total mononuclear cells. No immune response is seen and the tumor keeps growing to reach 1 square cm size. Mice are then injected intravenously either with
   a) 10 µg of pan tumor carcinoma antigen,
   b) with 0.1 mg of IgG anti-pan carcinoma Ag,
   c) with both,
   d) with 1 million activated autologous macrophages,
   e) with both Ag plus Ab and macrophages.

   The absence of objective response is seen in groups a), b), and c) ; partial transient response in groups d) and a tumor regression in group e) are documented by measurement of tumor size with calipets in two perpendicular directions.
2) Anti-HBV antibodies are mixed with HBs particles in a ratio 10/1 with about 10⁷ HBs/ml and incubated for 4h at 37°C in IMDM medium with about 10⁶/ml monocyte derived cells. The resulting monocyte derived cells of the invention induce the proliferation of cytotoxic T lymphocytes (autologous to the monocyte derived cells) specific for HBV (10⁶ monocyte derived cells resuspended per ml or RPMI medium added to 5.10⁵ T lymphocytes during 5 days, followed by measurement of tritiated thymidine incorporation).
3) The emergence of resistance to antibiotics in hospitals, even after triple therapies, requires the development of new vaccinal immunotherapies of infections. Monocyte derived cells obtained *ex vivo* are resuspended in culture medium in the presence a: of monoclonal or polyclonal antibodies specific for the resistant infectious agent (e.a. virus, staphylococci) or of anti-LPS antibodies for Gram negative bacteria, and b: in the presence of killed pathogens (pasteurisation) or membrane extracts.
   A ternary complex is formed between the infectious antigen, the antibodies and the monocyte derived cells. These monocyte derived cells cultured at about 5.10⁶/ml in IMDM medium process the antibody-infectious antigen complex during 4h incubation time at 37°C. The cells are centrifuged, resuspended in isotonic saline and injected locally or systemically to the patient to induce protective therapeutic immunity against the infectious agents that can be illustrated by the decrease in the infection titer and the presence of anti-infectious antibodies in peripheral blood. It is shown that in mice with lethal Gram(-) infections, the injection of macrophages having processed anti-LPS antibodies and heat killed bacteria results in immune protection of the animals.
   The above-mentioned experiments have been performed with different subsets of monocyte derived cells expressing immunoglobulin Fc receptors, and in particular with macrophages.
4) Monocyte-derived macrophages of HLA-A2 haplotype, are incubated with the S protein of Hepatitis B virus in the presence or not of an anti-HBS immunoglobulin which displays a high affinity for the FcγRI, CD64. After 4 hours of incubation, the macrophages are mixed with increasing numbers of an HLA-A2 restricted T cell clone specific for HBS. The stimulation of the T cell clone is assessed by measuring the secretion of cytokines, e.g. IFNγ. It is shown that in the presence of the anti-HBS antibody, the stimulation of the T cell clone is 100 fold higher than in absence of the immunoglobulin.

## Claims

1. Process for the preparation of monocyte derived cells presenting antigenic epitopes on their membranes after interiorization and processing of at least an antigen-antibody complex, formed between an antigen and an antibody, said antibody being exclusively a non-bispecific antibody, and said process comprising the following steps :
a) preparation of the monocytes derived cells according to the following method:
1) recovery of blood derived mononuclear cells directly from blood apheresis or from blood bag collection, followed if necessary by centrifugation, to eliminate a substantial part of red blood cells granulocytes and platelets, and collection of peripheral blood leukocytes ;
2) washing peripheral blood leukocytes obtained at the preceeding steps for instance by centrifugation to remove 90 % of platelets, red blood cells and debris to obtain mononuclear cells ;
3) resuspension of the total mononuclear cells obtained at the preceeding step in culture medium such as AIMV, RPMI or IMDM type at 10⁶ to 2.10⁷ cells/ml,
b) addition of antigens and antibodies to the monocyte derived cells obtained at the preceeding step to form a ternary complex between monocyte derived cells, an antigen and an antibody ;
c) incubation of said ternary complex for a time sufficient and at a temperature above 20°C to allow endocytosis into intracellular vacuoles of the monocyte derived cells and processing of the antigen-antibody complex, with said processing consisting of digestion of the antigen-antibody complex and association of the epitopes of the antigen resulting from the digestion with MHC molecules, to obtain monocyte derived cells presenting antigenic epitopes on their membranes.

2. Process for preparation of monocyte derived cells according to claim 1, wherein in the step of resuspension of the total mononuclear cell, the culture medium is completed by cytokines and / or autologous serum.

3. Process according to claims 1 or 2, wherein
said antibody is directed against a tumor or against an infectious agent,
with said antigen being fragments of tumor or of infectious agent, including membranes or tumor apoptotic bodies, or purified tumor or infectious antigens or is a recombinant tumor or infectious antigen.

4. Process according to claim 1 or 2, wherein in the step of addition, said antigens and antibodies are in the form of a complex.

5. Process according to anyone of claims 1 to 4, comprising the additional following step:
d) centrifugation of the monocyte derived cells presenting antigenic epitopes on their membranes, washing and resuspension, for instance in isotonic medium, to obtain a suspension of the above defined monocyte derived cells.

6. Process according to claim 5, wherein the step of centrifugation is followed by
e) freezing at temperature below or equal to -80°C aliquots of the above said suspension, with the addition of a cryopreservative.

7. Process according to claim 6, wherein the step of freezing is followed by
f) melting said above frozen aliquots at a temperature enabling to obtain a suspension of monocyte derived cells presenting antigenic epitopes on their membranes, for instance at 4°C, washing said suspension and resuspending it, for instance in an isotonic medium, to obtain a suspension of monocyte derived cells presenting antigenic epitopes on their membranes.

8. Process according to anyone of claims 1 to 7, wherein the antibodies are human or humanised IgG, IgA or preferably IgM for induction of primary immune response directed against tumor antigens or against infectious antigens, such as viral or bacterial antigens.

9. Process according to anyone of claims 1 to 7, wherein the antigens are purified tumor, viral or bacterial antigens such as polypeptides, glycopeptides, oligosacharides, or membrane fragments serving as complex antigens.

10. Process according to claims 8 or 9, wherein the antigens are pan tumor antigens present on different tumor types.

11. Ternary complex in a purified form, being 90% pure and no contaminants other than antibody and the tumor antigen or apoptotic bodies or infectious antigens being present, between monocytes derived cells, an antigen and an antibody
. with said antibody being exclusively a non-bispecific antibody and being directed against a tumor or against an infectious agent,
. with said antigen being fragments of tumor or of infectious agent including membranes or tumor apoptotic bodies, or purified tumor or infectious antigens or being recombinant tumor or infectious antigens, and
. with said antigen and said antibody being liable to form an antigen-antibody complex under appropriate conditions.

12. Process for the preparation of a ternary complex between monocyte derived cells, an antigen and an antibody according to claim 11, comprising the following steps :
a) preparation of monocyte derived cells according to the following method :
1) recovery of blood derived mononuclear cells directly from blood apheresis or from blood bag collection, followed if necessary by centrifugation, to eliminate a substantial part of red blood cells granulocytes and platelets, and collection of peripheral blood leukocytes ;
2) washing peripheral blood leukocytes obtained at the preceeding steps for instance by centrifugation to remove 90 % of platelets, red blood cells and debris to obtain mononuclear cells ;
3) resuspension of the total mononuclear cells obtained at the preceeding step in culture medium such as AIMV, RPMI or IMDM type at 10⁶ to 2.10⁷ cells/ml, possibly completed by cytokines and/or autologous serum, and culture for 5 to 10 days at 37°C under O₂/CO₂ atmosphere in hydrophobic gas permeable bags, to obtain monocyte derived cells and contaminating lymphocytes ;
b) addition of antigens and antibodies to the monocyte derived cells obtained at the preceding step to form of a ternary complex between monocyte derived cells, an antigen and an antibody.

13. Process according to claim 12, wherein said antigens and antibodies are either in the form of a complex or not.

14. Ternary complex between monocyte derived cells, an antigen and an antibody such as obtained according to claims 12 or 13.

15. Pharmaceutical composition containing as active substance a ternary complex according to claim 14, in association with a pharmaceutically acceptable vehicle.

16. Pharmaceutical composition according to claim 15, in the form of sterile injectable preparations.

17. Vaccine containing as active substance a ternary complex according to claim 14, in association with a pharmaceutically acceptable vehicle.

18. Use of a ternary complex according to claim 14, for the preparation of a medicament for treating cancer or infectious diseases.

## Patentansprüche

1. Verfahren zur Herstellung von monozytenabgeleiteten Zellen, die Antigenepitope auf ihren Membranen, nach Aufnahme und Verarbeitung von zumindest einem Antigen-Antikörper-Komplex, gebildet zwischen einem Antigen und einem Antikörper, präsentieren, wobei besagter Antikörper ausschließlich ein nicht bispezifischer Antikörper ist und besagtes Verfahren die folgenden Schritte umfasst:
a) Präparation der monozytenabgeleiteten Zellen gemäß dem folgenden Verfahren:
1) Erhalten von blutabgeleiteten mononuklearen Zellen direkt durch Blutapherese oder durch Blutbeutelentnahme, wenn notwendig gefolgt von Zentrifugation, um einen wesentlichen Teil der roten Blutzellen, Granulozyten und Blutplättchen zu entfemen, und Sammeln von peripheren Blutleukozyten;
2) Waschen der peripheren Blutleukozyten, die durch den vorhergehenden Schritt erhalten wurden, z. B. durch Zentrifugation, um 90 % der Blutplättchen, roten Blutzellen und Debris zu entfemen, um mononukleare Zellen zu erhalten;
3) Resuspension der gesamten durch den vorhergehenden Schritt erhaltenen mononuklearen Zellen, in Kultivierungsmedium wie z. B. AIMV, RPMI oder IMDM Typ bei 10⁶ bis 2.10⁷ Zellen/ml,
b) Zugabe von Antigenen und Antikörpern zu den monozytenabgeleiteten Zellen, die bei dem vorhergehenden Schritt erhalten wurden, um einen ternären Komplex zwischen monozytenabgeleiteten Zellen, einem Antigen und einem Antikörper zu bilden;
c) Inkubation von besagtem ternären Komplex für eine ausreichende Zeit und bei einer Temperatur über 20 °C, um Endozytose in intrazelluläre Vakuolen der monozytenabgeleiteten Zellen und die Prozessierung des Antigen-Antikörper-Komplexes zu erlauben, wobei besagte Prozessierung aus der Verdauung des Antigen-Antikörper-Komplexes und der Assoziation der Epitope des Antigens, resultierend aus der Verarbeitung mit MHC Molekülen besteht, um monozytenabgeleitete Zellen, die Antigenepitope auf ihren Membranen präsentieren zu erhalten.

2. Verfahren für die Herstellung von monozytenabgeleiteten Zellen nach Anspruch 1, wobei bei dem Schritt der Resuspension der gesamten mononuklearen Zellen, das Kultivierungsmedium durch Zytokine und/oder autologes Serum vervollständigt ist.

3. Verfahren nach Anspruch 1 oder 2, wobei besagter Antikörper gegen einen Tumor oder gegen ein infektiöses Agenz gerichtet ist, und wobei besagte Antigene Fragmente von Tumor oder von infektiösem Agenz sind, beinhaltend Membranen oder tumorapoptotische Körper, oder gereinigte Tumor- oder infektiöse Antigene oder ein rekombinantes Tumor- oder infektiöses Antigen sind.

4. Verfahren nach Anspruch 1 oder 2, wobei bei dem Schritt der Zugabe, besagte Antigene und Antikörper in der Form eines Komplexes vorliegen.

5. Verfahren nach einem der Ansprüche 1 bis 4, umfassend den zusätzlichen folgenden Schritt:
d) Zentrifugation der monozytenabgeleiteten Zellen, die Antigencpitopc auf ihren Membran präsentieren, Waschen und Resuspension, z. B. in isotonischem Medium, um eine Suspension der oben beschriebenen monvzytenabgeleiteten Zellen zu erhalten.

6. Verfahren nach Anspruch 5, wobei der Schritt der Zentrifugation gefolgt wird von
c) Einfrieren von Aliquoten der oben genannten Suspension bei Temperaturen unter oder gleich -80 °C, mit der Zugabe von einem Kühlschutzmittel.

7. Verfahren nach Anspruch 6, wobei der Schritt des Einfrierens gefolgt wird von
f) Auftauen besagter oben gefrorener Aliquote bei einer Temperatur, die es erlaubt eine Suspension von monozytenabgeleiteten Zellen, die Antigenepitope auf ihren Membranen präsentieren, zu erhalten, z. B. bei 4 °C, Waschen besagter Suspension und Resuspension davon, z. B. in einem isotonischen Medium, um eine Suspension von monozytenabgeleiteten Zellen, die Antigenepitopc auf ihren Membranen präsentieren zu erhalten.

8. Verfahren nach einem der Ansprüche 1 bis 7, wobei die Antikörper menschliches oder humanisiertes IgG, IgA oder bevorzugt IgM, für die Induktion einer primären Immunantwort, gerichtet gegen Tumorantigene oder gegen infektiöse Antigene, wie z. B. virale oder bakterielle Antigene sind.

9. Verfahren nach einem der Ansprüche 1 bis 7, wobei die Antigene gereinigte Tumor-, virale oder bakterielle Antigene, wie z. B. Polypeptide, Glykopeptide, Oligosacharide oder Membranfragmente sind, die als Komplexantigene dienen.

10. Verfahren nach Anspruch 8 oder 9, wobei die Antigene Pantumor-Antigene, vorhanden bei verschiedenen Tumortypen sind.

11. Ternärer Komplex in einer gereinigten Form, der 90 % rein ist und wo keine Kontaminanten, außer den Antikörpern und den Tumorantigenen oder apoptotischen Körperchen oder infektiösen Antigenen vorhanden sind, zwischen monozytenabgeleitete Zellen, einem Antigen und einem Antikörper,
• wobei besagter Antikörper ausschließlich ein nicht bispezifischer Antikörper ist und gegen einen Tumor oder gegen ein infektiöses Agenz gerichtet ist,
• und wobei besagtes Antigen Fragmente eines Tumors oder eines infektiösen Agenz, beinhaltend Membranen oder tumorapoptotische Körper, oder gereinigtes Tumor- oder infektiöses Antigen ist, oder rekombinantes Tumor- oder infektiöses Antigen ist, und
• wobei besagtes Antigen und besagter Antikörper geeignet sind einen Antigen-Antikörper-Komplex unter geeigneten Bedingungen zu bilden.

12. Verfahren für die Herstellung eines ternären Komplexes zwischen monozytenabgeleiteten Zellen, einem Antigen und einem Antikörper nach Ansprach 11, umfassend die folgenden Schritte:
a) Präparation der monozytenabgeleiteten Zellen gemäß dem folgenden Verfahren:
1) Erhalten von blutabgeleiteten mononuklearen Zellen direkt durch Blutapherese oder durch Blulbeutelentnahme, wenn notwendig gefolgt von Zentrifugation, um einen wesentlichen Teil der roten Blutzellen, Granulozyten und Blutplättchen zu entfemen, und Sammeln von peripheren Blutleukozyten;
2) Waschen der peripheren Blutleukozyten, die durch den vorhergehenden Schritt erhalten wurden, z. B. durch Zentrifugation, um 90 % der Blutplättchen, roten Blutzellen und Debris zu entfernen, um mononukleare Zellen zu erhalten;
3) Resuspension der gesamten durch den vorhergehenden Schritt erhaltenen mononuklearcn Zellen, in Kultivierungsmedium wie z. B. AIMV, RPMT oder IMDM Typ bei 10⁶ bis 2.10⁷ Zellen/ml, eventuell vervollständigt durch Zytokine und/oder autologes Serum und Kultivierung für 5 bis 10 Tage bei 37 °C unter einer O₂/CO₂ Atmosphäre in hydrophoben, gasdurchlässigen Beuteln, um monozytenabgeleitete Zellen und kontaminicrende Lymphoxyten zu erhalten;
b) Zugabe von Antigenen und Antikörpern zu den monozytenabgeleiteten Zellen, erhalten bei dem vorhergehenden Schritt, um einen ternären Komplex zwischen monozytenabgeleiteten Zellen, einem Antigen und einem Antikörper zu bilden.

13. Verfahren nach Anspruch 12, wobei besagte Antigene und Antikörper entweder in der Form eines Komplexes sind oder nicht.

14. Ternärer Komplex zwischen monozytenabgeleiteten Zellen, einem Antigen und einem Antikörper, wie erhalten nach Ansprüchen 12 oder 13.

15. Pharmazeutische Zusammensetzung beinhaltend als aktive Substanz einen ternären Komplex nach Anspruch 14 in Verbindung mit einem pharmazeutisch akzeptablen Träger.

16. Pharmazeutische Zusammensetzung nach Anspruch 15, in der Form eines sterilen, injizierbaren Präparats.

17. Impfstoff beinhaltend als aktive Substanz einen ternären Komplex nach Anspruch 14, in Verbindung mit einem pharmazeutisch akzeptablen Träger.

18. Verwendung eines ternären Komplexes nach Anspruch 14, für die Herstellung eines Medikaments zur Behandlung von Krebs oder infektiösen Krankheiten.

## Revendications

1. Procédé de préparation de cellules dérivées de monocytes présentant des épitopcs antigéniques sur leurs membranes après intériorisation et protéolyse d'au moins un complexe antigène-anticorps, formé entre un antigène et un anticorps, ledit anticorps étant exclusivement un anticorps non bispécifique, et ledit procédé comprenant les étapes suivantes :
a) la préparation de cellules dérivées de monocytes selon la méthode suivante :
1) la récupération de cellules mononuclées dérivées de sang directement à partir d'une aphérèse sanguine ou à partir d'une collecte de poches de sang, suivie si nécessaire par une centrifugation, pour éliminer une partie substantielle des hématies, des granulocytes et des plaquettes, et collecte des leucocytes du sang périphérique.
2) le lavage des leucocytes du sang périphérique obtenus aux étapes précédentes, par exemple par centrifugation, pour éliminer 90% des plaquettes, des hématies et des débris, pour obtenir des cellules mononuclées ;
3) la resuspension de la totalité des cellules mononuclées obtenues à l'étape précédente dans un milieu de culture tel que AIMV, RPMI ou le type IMDM à 10⁶ à 2.10⁷ cellules/ml,
b) l'addition d'antigènes et d'anticorps aux cellules dérivées de monocytes obtenues à l'étape précédente pour former un complexe ternaire entre les cellules dérivées de monocytes, un antigène et un anticorps ;
c) l'incubation dudit complexe ternaire pendant un temps suffisant et à une température supérieure à 20°C pour permettre l'endocytose des cellules dérivées de monocytes dans des vacuoles intracellulaires et la protéolyse du complexe antigène-anticorps, ladite protéolyse consistant en la digestion du complexe antigène-anticorps et en l'association des épitopes de l'antigène résultant de la digestion avec des molécules du CMH, pour obtenir des cellules dérivées de monocytes présentant des épitopes antigéniques sur leurs membranes.

2. Procédé de préparation de cellules dérivées de monocytes selon la revendication 1, dans lequel dans l'étape de resuspension de la totalité des cellules mononuclées, le milieu de culture est complété par des cytokines et / ou un sérum autologue.

3. Procédé selon la revendication 1 ou 2, dans lequel ledit anticorps est dirigé contre une tumeur ou contre un agent infectieux, ledit antigène étant des fragments de tumeur ou d'agent infectieux, incluant des corps apoptotiques de membrane ou de tumeur, ou des antigènes infectieux ou tumoraux purifiés ou étant un antigène infectieux ou tumoral recombinant.

4. Procédé selon la revendication 1 ou 2, dans lequel dans l'étape d'addition, lesdits antigènes et anticorps sont sous la forme d'un complexe.

5. Procédé selon l'une quelconque des revendications 1 à 4, comprenant l'étape supplémentaire suivante :
d) la centrifugation des cellules dérivées de monocytes présentant des épitopes antigéniques sur leurs membranes, le lavage et la resuspension, par exemple dans un milieu isotonique, pour obtenir une suspension de cellules dérivées de monocytes définies ci-dessus.

6. Procédé selon la revendication 5, dans lequel l'étape de centrifugation est suivie de
c) la congélation d'aliquotes de ladite suspension ci-dessus à une température inférieure ou égale a -80°C, avec l'addition d'un cryocunservateur.

7. Procédé selon la revendication 6, dans lequel l'étape de congélation est suivie de
f) la décongélation desdits aliquotes congelés ci-dessus à une température permettant d'obtenir une suspension de cellules dérivées de monocytes présentant des épitopes antigéniques sur leur membranes, par exemple à 4°C, le lavage de ladite suspension et la resuspension de celle-ci, par exemple dans un milieu isotonique, pour obtenir une suspension de cellules dérivées de monocytes présentant des épitopes antigéniques sur leur membranes.

8. Procédé selon l'une quelconque des revendications 1 à 7, dans lequel les anticorps sont des IgG, IgA ou, de préférence, IgM humaines ou humanisées, pour induire une réponse immunitaire primaire, dirigés contre des antigènes tumoraux ou contre des antigènes infectieux, tels que les antigènes viraux ou bactériens.

9. Procédé selon l'une quelconque des revendications 1 à 7, dans lequel les antigènes sont des antigènes tumoraux, viraux ou bactériens purifiés tels que des polypeptides, des glycopeptides, des oligosaccharides ou des fragments membranaires servant d'antigènes complexes.

10. Procédé selon la revendication 8 ou 9, dans lequel les antigènes sont des antigènes tumoraux pan présents sur différents types de tumeurs.

11. Complexe ternaire sous une forme purifiée, pur à 90% et sans aucun contaminant autre qu'un anticorps et l'antigène tumoral ou des corps apoptotiques ou des antigènes infectieux, entre les cellules dérivées de monocytes, un antigène et un anticorps
• ledit anticorps étant exclusivement un anticorps non bispécifique et étant dirigé contre une tumeur ou contre un agent infectieux,
• ledit antigène étant des fragments de tumeur ou d'agent infectieux incluant des corps apoptotiques membranaires ou tumoraux, ou des antigènes tumoraux ou infectieux purifiés ou étant des antigènes tumoraux ou infectieux recombinants, et
• ledit antigène et ledit anticorps étant capables de former un complexe antigène-anticorps dans des conditions appropriées.

12. Procédé de préparation d'un complexe ternaire entre des cellules dérivées de monocytes, un antigène et un anticorps selon la revendication 11, comprenant les étapes suivantes :
a) la préparation de cellules dérivées de monocytes selon la méthode suivante :
1) la récupération de cellules mononuclées dérivées du sang directement à partir d'une aphérèse sanguine ou d'une collecte de poches de sang, suivie si nécessaire d'une centrifugation, pour éliminer une partie substantielle des hématies, des granulocytes et des plaquettes, et collecte de leucocytes du sang périphérique ;
2) le lavage des leucocytes du sang périphérique obtenus aux étapes précédentes par exemple par centrifugation, pour éliminer 90% des plaquettes, des hématies et des débris, pour obtenir des cellules mononuclées;
3) la resuspension de la totalité des cellules mononuclées obtenues à l'étape précédente dans un milieu de culture tel que AIMV, RPMI ou le type IMDM à 10° à 2.10⁷ cellules/ml, éventuellement complétée par des cytokines et/ou un sérum autologue, et la culture pendant 5 à 10 jours à 37°C sous atmosphère O₂/Co2 dans des sacs hydrophobes pcrméables au gaz, pour obtenir des cellules dérivées de monocytes et des lymphocytes contaminants;
b) l'addition d'antigènes et d'anticorps aux cellules dérivées de monocytes obtenues à l'étape précédente pour former un complexe ternaire entre des cellules dérivées de monocytes, un antigène et un anticorps.

13. Procédé selon la revendication 12, dans lequel lesdits antigènes et anticorps sont sous la forme d'un complexe ou ne sont pas sous la forme d'un complexe.

14. Complexe ternaire entre des cellules dérivées de monocytes, un antigène et un anticorps tel qu'obtenu selon la revendication 12 ou 13.

15. Composition pharmaceutique contenant comme substance active un complexe ternaire selon la revendication 14, en association avec un excipient pharmaceutiquement acceptable.

16. Composition pharmaceutique selon la revendication 15, sous forme de préparations injectables stériles.

17. Vaccin contenant comme substance active un complexe ternaire selon la revendication 14, en association avec un excipient pharmaceutiquement acceptable.

18. Utilisation d'un complexe ternaire selon la revendication 14, pour la préparation d'un médicament pour traiter le cancer ou des maladies infectieuses.
